# EUROPEAN PATENT APPLICATION

(11) **EP 4 344 708 A1**
(43) Date of publication of application: **03.04.2024**
(21) Application number: 22210792.2
(22) Date of filing: 01.12.2022
(51) Int. Cl.: A61L 2/07, A61J 9/00, F16K 31/00

(54) **APPARATUS AND METHOD FOR STERILIZING INFANT FEEDING EQUIPMENT**

(30) Priority: 30.09.2022 WO PCT/CN2022/123364
(71) Applicant: Koninklijke Philips N.V., 5656 AG Eindhoven (NL)
(72) Inventor: XU, Changguan, Eindhoven (NL); LUI, Kwan Fai, Eindhoven (NL)
(74) Representative: Philips Intellectual Property & Standards

(57) **Abstract**

Provided is an infant feeding equipment-sterilizing apparatus (100). The apparatus comprises a chamber (102, 104) for receiving infant feeding equipment to be steam sterilized. A vent (108) is provided for venting air and/or steam from the chamber, e.g. to atmosphere. A valve assembly (110) restricts venting via the vent during steam sterilizing and allows venting from the chamber via the vent following the steam sterilizing. The venting being allowed following the steam sterilizing may assist to dry the infant feeding equipment following the steam sterilizing. However, by the valve assembly restricting, e.g. blocking, venting from the chamber via the vent during steam sterilizing, more of the steam supplied into the chamber can be retained therein during the steam sterilizing, thereby assisting to make the steam sterilization more energy efficient. Further provided is a chamber wall portion, e.g. lid, for an infant feeding equipment-sterilizing apparatus, and a method for sterilizing infant feeding equipment using such an apparatus.

## Description

### FIELD OF THE INVENTION

This invention relates to an infant feeding equipment-sterilizing apparatus, and a method of sterilizing infant feeding equipment using such an apparatus.

### BACKGROUND OF THE INVENTION

Electric sterilizers are popular among parents of infants for sterilizing infant feeding equipment, for example baby milk bottles, teats, and accessories, in a relatively safe and rapid manner, which avoids the use of chemicals.

The infant feeding equipment to be sterilized may be placed in a basket included in the sterilizer, and a lid placed on top of the basket assists to prevent excessive hot air leakage and to retain heat within the basket.

During sterilizing, a heating element in a base unit on which the basket is placed vaporizes water and generates hot steam that is delivered into the basket to sterilize the infant feeding equipment.

It may be desirable to dry the infant feeding equipment following the sterilization. To this end, vents, in other words exhaust holes, provided in the lid can allow residual humidity to escape from the basket after sterilizing. Whilst such vents can assist to speed up the drying process, steam and hot air can also escape from basket via the vents during sterilizing. This can result in a longer heating time being required for the sterilizing, and thus more energy may be consumed/wasted during the sterilizing. Moreover, such escape of steam from the basket via the vents can mean that more water is consumed, and can provide an unnecessarily large contribution to humidity in the room in which the sterilizer is being used.

### SUMMARY OF THE INVENTION

The invention is defined by the claims.

According to examples in accordance with an aspect of the invention, there is provided an infant feeding equipment-sterilizing apparatus, the apparatus comprising: a chamber for receiving infant feeding equipment to be steam sterilized; a vent for venting air and/or steam from the chamber; and a valve assembly switchable between restricting venting from the chamber via the vent during steam sterilizing, and allowing venting from the chamber via the vent following the steam sterilizing.

By the valve assembly allowing venting from the chamber following the steam sterilizing, the steam-sterilized infant feeding equipment may be assisted to dry more rapidly. This is because residual humidity present in the chamber may be vented and/or condensed water on the infant feeding equipment may be allowed to evaporate and escape from the chamber via the vent.

However, by the valve assembly restricting, e.g. blocking, venting from the chamber via the vent during the steam sterilizing, more of the steam supplied into the chamber may be retained therein during the steam sterilizing. This may assist to make the steam sterilization more energy efficient.

The switching of the valve assembly between restricting venting from the chamber via the vent during steam sterilizing, and allowing venting from the chamber via the vent following the steam sterilizing is, for example, automatic.

A steam supply may supply steam into the chamber in order to implement the steam sterilizing.

In some embodiments, the apparatus comprises such a steam supply in the form of a steam generator.

The vent may be provided in a chamber wall portion that at least partly delimits the chamber.

In some embodiments, the chamber wall portion is a lid openable to access the chamber.

In such embodiments, the lid may be moveably, e.g. detachably, mounted to a housing, with the chamber being enclosed by the lid and the housing when the lid is in place to close the chamber.

The vent and at least part of the valve assembly can, for example, be provided in the lid.

Alternatively or additionally, the valve assembly may be arranged at or proximal to the top of the chamber, for example by being included in a top lid that closes the chamber from above.

It is noted that other positions for the valve assembly can also be contemplated, e.g. in relation to airflow in the chamber.

In at least some embodiments, the vent defines the only passage for air and/or steam out of the chamber.

In such embodiments, the risk of excessive pressure build-up inside the chamber during steam sterilizing may be mitigated by limiting the amount of steam being generated and employing a chamber having a sufficiently large volume. Limiting the amount of steam being generated may, for instance, involve limiting a capacity of a water holder used to retain water used to generate the steam, e.g. to at most 130 mL.

Alternatively or additionally, in embodiments in which the apparatus includes the lid, no mechanical locking and sealing member may be arranged between the lid and the housing, such that the risk of excessive pressure in the chamber may be reduced via the possibility of gas escaping between the lid and the housing.

The valve assembly restricting and allowing venting from the chamber via the vent respectively during and following the steam sterilizing can be implemented in any suitable manner.

In some embodiments, one or more conditions following the steam sterilizing is or are different from during the steam sterilizing, and the valve assembly is configured to respond to the one or more different conditions following the steam sterilizing to switch from restricting to allowing venting from the chamber via the vent.

In some embodiments, the atmospheric conditions within the chamber cause the valve assembly to switch.

Such atmospheric conditions may, for example, include one or more of temperature, pressure, humidity, and air movement.

Alternatively or additionally, electrical or electromechanical means that are used to create a change in the chamber cause switching of the valve assembly.

For example, turning of a blower, e.g. ventilator, shaft may open a valve of the valve assembly.

It is noted that the air movement caused by the blower, e.g. ventilator, may be an overlapping point with the above-mentioned air movement-driven valve switching.

Alternatively or additionally, a mechanical switch may switch off the steam and switch on the heater and/or blower, and this action, e.g. caused by user interaction with the apparatus, may cause a valve of the valve assembly to open.

In a simple embodiment, a switch actuated by a user causes opening of a valve of the valve assembly.

In some embodiments, a temperature in the chamber during the steam sterilizing mode is different from, for example higher than, a temperature in the chamber following operation in the steam sterilizing mode. In such embodiments, the valve assembly may be configured to respond to the temperature in the chamber to switch between restricting and allowing of venting from the chamber via the vent.

Thus, the different, e.g. lower, temperature employed following the steam sterilizing may cause the valve assembly to switch from restricting venting from the chamber via the vent to allowing venting from the chamber via the vent.

In some embodiments, the valve assembly comprises a thermally responsive deformable member whose thermally responsive deformation enables the switching between restricting and allowing of venting from the chamber via the vent.

In at least some embodiments, the deformation of the thermally responsive deformable member is reversible such as to enable repeated switching between restricting and allowing of venting from the chamber via the vent.

Any suitable type of thermally responsive deformable member can be contemplated provided that the thermally responsive deformable member is capable of deforming, e.g. reversibly deforming, to enable switching between restricting and allowing of venting from the chamber via the vent.

In some embodiments, the thermally responsive deformable member comprises a layered strip having a first layer and a second layer arranged on the first layer, with a coefficient of thermal expansion of the first layer being different to that of the second layer such that the layered strip provides the thermally responsive deformation.

The layered strip can, for example, comprise or be a bimetallic strip.

Such a bimetallic strip may have the advantage of exhibiting relatively large deformation, particularly in a relatively small area, in response to temperature change.

In some embodiments, the vent comprises an aperture delimited by the chamber wall portion, e.g. an aperture in a lid defining the chamber wall portion, and the valve assembly comprises a valve seat that extends around the aperture.

In such embodiments, the valve seat may comprise a sealing member, such as an elastomeric sealing member.

Such a sealing member, e.g. elastomeric sealing member, may assist to retain steam within the chamber during the steam sterilizing.

In embodiments in which the valve assembly comprises the thermally responsive deformable member, the thermally responsive deformable member may releasably engage the sealing member to restrict and allow venting from the chamber via the vent.

In some embodiments, the thermally responsive member comprises the layered strip, e.g. bimetallic strip, and the sealing member is an elastomeric sealing member, e.g. formed from silicone rubber.

This has been found to provide a particularly straightforwardly implementable and effective combination for realizing the valve assembly.

In some embodiments, the apparatus includes a cover member arranged over the thermally responsive deformable member, e.g. layered strip, with a passage for the released air and/or steam being defined between the cover member and an exterior surface of the chamber wall portion that at least partly delimits the chamber.

Such a cover member may assist to protect the thermally responsive deformable member, e.g. layered strip, from being polluted, for instance by dust.

In some embodiments, the apparatus includes a switching assembly for switching the apparatus between operation in a steam sterilizing mode and operation in an equipment drying mode in which the supply of steam into the chamber is restricted or ceased.

In such embodiments, the valve assembly may be configured to, in response to switching via the switching assembly, switch between restricting and allowing of venting from the chamber via the vent.

In at least some embodiments, the apparatus includes a blower configured to, while the valve assembly is allowing venting from the chamber via the vent, blow steam and/or air from the chamber via the vent.

The blower may assist to dry the infant feeding equipment following the steam sterilization.

In such embodiments, the equipment drying mode may comprise operating the blower to blow steam and/or air from the chamber via the vent.

The blower may have any suitable design. In some embodiments, the blower comprises a motor and a fan, whose rotation to displace steam and/or air from the chamber via the vent, is driven by the motor.

In some embodiments, the valve assembly is configured to, responsive to operation of the blower, allow venting from the chamber via the vent.

Thus, operation of the blower may trigger the valve assembly to allow venting of air and/or steam from the chamber via the vent.

In embodiments in which the apparatus includes the blower, a pressure in the chamber during the steam sterilizing may be different from, e.g. lower than, a pressure in the chamber following the steam sterilizing due to operation of the blower to dry the infant feeding equipment following but not during the steam sterilizing.

In such embodiments, the valve assembly may be configured to respond to the pressure in the chamber to switch between restricting and allowing of venting from the chamber via the vent.

Alternatively or additionally, the valve assembly may be configured to respond to an airflow in the chamber, e.g. an airflow direction, position and/or magnitude, that is different following the sterilizing as compared to during the sterilizing to switch between restricting and allowing of venting from the chamber via the vent.

In embodiments in which the apparatus includes the switching assembly, the switching assembly may include or be defined by a solenoid, e.g. a solenoid arranged to activate the motor to rotate the fan of the blower.

In some embodiments, the valve assembly comprises a valve member connected to a linking member that is arranged to transfer movement from the switching assembly, e.g. solenoid, to the valve member in order to open and close the vent in response to activation of the motor to rotate the fan of the blower.

Alternatively or additionally, movement of the motor may cause the valve assembly to switch between restricting and allowing of venting from the chamber via the vent.

According to another aspect there is provided a chamber wall portion for an infant feeding equipment-sterilizing apparatus, the chamber wall portion being for at least partly delimiting a chamber in which infant feeding equipment to be steam sterilized is receivable, wherein a vent for venting air and/or steam from the chamber is provided in the chamber wall portion together with at least part of a valve assembly that is switchable between restricting venting from the chamber via the vent during steam sterilizing, and allowing venting from the chamber via the vent following the steam sterilizing.

By the valve assembly allowing venting from the chamber following the steam sterilizing, the steam-sterilized infant feeding equipment may be assisted to dry more rapidly. This is because residual humidity present in the chamber may be vented and/or condensed water on the infant feeding equipment may be allowed to evaporate and escape from the chamber via the vent.

However, by the valve assembly restricting, e.g. blocking, venting from the chamber via the vent during the steam sterilizing, more of the steam supplied into the chamber may be retained therein during the steam sterilizing. This may assist to make the steam sterilization more energy efficient.

Thus, the chamber wall portion of this aspect provides an alternative solution to the same problem described above in relation to the infant feeding equipment-sterilizing apparatus.

In some embodiments, the chamber wall portion is a lid for the infant feeding equipment-sterilizing apparatus.

In such embodiments, the lid may be moveably, e.g. detachably, mountable to a housing of the infant feeding equipment-sterilizing apparatus, with the chamber being enclosed by the lid and the housing when the lid is in place to close the chamber.

The switching of the valve assembly between restricting venting from the chamber via the vent during steam sterilizing, and allowing venting from the chamber via the vent following the steam sterilizing is, for example, automatic.

In some embodiments, one or more conditions following the steam sterilizing is or are different from during the steam sterilizing, and the valve assembly is configured to respond to the one or more different conditions following the steam sterilizing to switch from restricting to allowing of venting from the chamber via the vent.

In some embodiments, a temperature in the chamber during the steam sterilizing mode is different from, for example higher than, a temperature in the chamber following operation in the steam sterilizing mode. In such embodiments, the valve assembly may be configured to respond to the temperature in the chamber to switch between restricting and allowing of venting from the chamber via the vent.

In some embodiments, the valve assembly comprises a thermally responsive deformable member whose thermally responsive deformation enables the switching between restricting and allowing of venting from the chamber via the vent.

In at least some embodiments, the deformation of the thermally responsive deformable member is reversible such as to enable repeated switching between restricting and allowing of venting from the chamber via the vent.

In some embodiments, the thermally responsive deformable member comprises a layered strip having a first layer and a second layer arranged on the first layer, with a coefficient of thermal expansion of the first layer being different to that of the second layer such that the layered strip provides the thermally responsive deformation.

The layered strip can, for example, comprise or be a bimetallic strip.

In some embodiments, the entirety of the valve assembly is provided in the chamber wall portion.

According to a further aspect there is provided a method for sterilizing infant feeding equipment using an apparatus having a chamber, a vent for venting air and/or steam from the chamber, and a valve assembly, the method comprising: receiving infant feeding equipment in the chamber; and steam sterilizing the infant feeding equipment, wherein venting from the chamber via the vent is restricted by the valve assembly during the steam sterilizing, and is allowed by the valve assembly following the steam sterilizing.

In some embodiments, the method further comprises, following the steam sterilizing, drying the infant feeding equipment, with the drying the infant feeding equipment comprising restricting or ceasing steam supply into the chamber while the valve assembly is allowing venting from the chamber via the vent.

In some embodiments, the apparatus further comprises a blower, and the drying the infant feeding equipment comprises operating the blower to blow steam and/or air from the chamber via the vent.

In some embodiments, one or more conditions following the steam sterilizing is or are different from during the steam sterilizing, and the valve assembly is configured to respond to the one or more different conditions following the steam sterilizing to switch from restricting to allowing of venting from the chamber via the vent.

In some embodiments, a temperature in the chamber during the steam sterilizing mode is different from, for example higher than, a temperature in the chamber following operation in the steam sterilizing mode. In such embodiments, the valve assembly may be configured to respond to the temperature in the chamber to switch between restricting and allowing of venting from the chamber via the vent.

In some embodiments, the valve assembly comprises a thermally responsive deformable member whose thermally responsive deformation enables the switching between restricting and allowing of venting from the chamber via the vent.

In at least some embodiments, the deformation of the thermally responsive deformable member is reversible such as to enable repeated switching between restricting and allowing of venting from the chamber via the vent.

In some embodiments, the thermally responsive deformable member comprises a layered strip having a first layer and a second layer arranged on the first layer, with a coefficient of thermal expansion of the first layer being different to that of the second layer such that the layered strip provides the thermally responsive deformation.

The layered strip can, for example, comprise or be a bimetallic strip.

More generally, embodiments described herein in relation to the method may be applicable to the apparatus and the chamber wall portion, embodiments described herein in relation to the apparatus may be applicable to the method and the chamber wall portion, and embodiments described herein in relation to the chamber wall portion may be applicable to the apparatus and the method.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
FIG. 1 provides a cross-sectional view of an apparatus according to an example;
FIG. 2A provides an enlarged view of a vent and valve assembly included in the apparatus shown in FIG. 1 during steam sterilizing;
FIG. 2B provides an enlarged view of the vent and valve assembly included in the apparatus shown in FIG. 1 following steam sterilizing; and
FIG. 3 provides a cross-sectional view of an apparatus according to another example.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Provided is an infant feeding equipment-sterilizing apparatus. The apparatus comprises a chamber for receiving infant feeding equipment to be steam sterilized. A vent is provided for venting air and/or steam from the chamber, e.g. to atmosphere. A valve assembly restricts venting via the vent during steam sterilizing and allows venting from the chamber via the vent following the steam sterilizing. The venting being allowed following the steam sterilizing may assist to dry the infant feeding equipment following the steam sterilizing. However, by the valve assembly restricting, e.g. blocking, venting from the chamber via the vent during steam sterilizing, more of the steam supplied into the chamber can be retained therein during the steam sterilizing, thereby assisting to make the steam sterilization more energy efficient. Further provided is a chamber wall portion, e.g. lid, for an infant feeding equipment-sterilizing apparatus, and a method for sterilizing infant feeding equipment using such an apparatus.

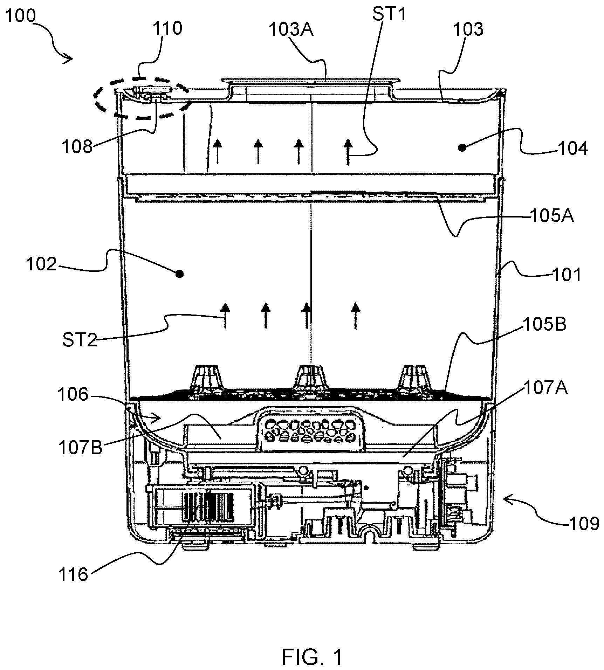

FIG. 1 shows an apparatus 100 according to an example. The apparatus 100 is configured as an apparatus 100 that sterilizes infant feeding equipment, such as one or more infant milk bottles, teat(s), screw ring(s) for securing a teat to an infant milk bottle, teat cover(s), pacifier(s), and/or other small accessories (not visible). Thus, the apparatus 100 can be regarded as an infant feeding equipment-sterilizing apparatus 100.

The apparatus 100 may include a chamber wall portion 103 that at least partly delimits a chamber 102, 104 in which the infant feeding equipment is receivable.

Once received in the chamber 102, 104, the infant feeding equipment may be sterilized using steam.

In some embodiments, such as that shown in FIG. 1, the chamber wall portion 103 is a lid openable to access the chamber 102, 104.

In such embodiments, the lid may be moveably, e.g. detachably, mounted to a housing 101, with the chamber 102, 104 being enclosed by the lid and the housing 101 when the lid is in place to close the chamber 102, 104.

Such a lid may, for example, include a handle 103A to assist with moving the lid to open and close the chamber 102, 104.

In embodiments in which the lid is a top lid, when the apparatus 100 is orientated for use, the lid may assist to minimize hot air escape and retain heat in the chamber 102, 104.

The chamber wall portion 103, e.g. lid, and the housing 101 can be formed of any suitable material. In some embodiments, the wall portion 103, e.g. lid, and/or the housing 101 is or are made of an engineering thermoplastic, such as polypropylene.

In certain embodiments, such as that shown in FIG. 1, the chamber 102, 104 is partitioned by a perforate dividing wall 105A.

Such partitioning can assist the user with arranging/organizing various pieces of infant feeding equipment in the chamber 102, 104. Moreover, hole(s) in the perforate dividing wall 105A mean that steam used for sterilizing the feeding equipment can reach the infant feeding equipment by passing through the hole(s).

Such a perforate dividing wall 105A can, for example, partition the chamber 102, 104 into a lower chamber portion 102 beneath the perforate dividing wall 105A when the apparatus 100 is orientated for use, with an upper chamber portion 104 being above the perforate dividing wall 105A.

Thus, steam may rise through the hole(s) in the perforate dividing wall 105A from the lower chamber portion 102 into the upper chamber portion 104. Steam rising in the upper chamber portion 104 is represented in FIG. 1 by the arrows ST1.

In such embodiments, the hole(s) in the perforate dividing wall 105A may be small enough so that pieces of infant feeding equipment, such as infant milk bottle(s), teat(s), screw ring(s), teat cover(s) and/or pacifier(s), may be supported on the perforate dividing wall 105A rather than falling through the hole(s).

In alternative embodiments, there is no perforate dividing wall 105A partitioning the chamber 102, such that the chamber 102 is defined by a single chamber portion.

A steam supply 106 may supply steam into the chamber 102, 104 in order to implement the steam sterilizing.

The steam supply 106 can be arranged to supply steam into the chamber 102, 104 in any suitable manner.

In some embodiments, such as that shown in FIG. 1, the apparatus 100 comprises a steam supply 106 in the form of a steam generator.

In such embodiments, the chamber 102, 104 may be partly delimited by a perforate wall 105B whose opening(s) permit steam from the steam generator to enter the chamber 102, 104.

The perforate wall 105B can be regarded as being included in a basket of the apparatus 100, with the interior of the basket defining the chamber 102, 104.

The perforate wall 105B can, for instance, be a perforate base of the chamber 102, 104 on which pieces of infant feeding equipment are supportable. In such embodiments, the opening(s) in the perforate base may be small enough so that pieces of infant feeding equipment, such as infant milk bottle(s), teat(s), screw ring(s), teat cover(s) and/or pacifier(s), may be supported on the perforate base rather than falling through the opening(s).

The steam generator may include a heating element 107A arranged to vaporize water contained in a water holder 107B adjacent the heating element 107A.

In some embodiments, the steam generator may be arranged beneath the perforate wall 105B when the apparatus 100 is orientated for use, such that steam generated in the steam generator can rise through the opening(s) in the perforate wall 105B and into the chamber 102, 104.

It is noted that the arrows ST2 in FIG. 1 represent steam generated by the steam generator that is rising in the chamber 102, 104, and in particular in the lower chamber portion 102 of the chamber 102, 104 in the depicted non-limiting example.

In some embodiments, the apparatus 100 comprises a base unit 109, with the heating element 107A being included, e.g. mounted, in the base unit 109.

In such embodiments, the water holder 107B may also be included in the base unit 109.

In some embodiments, the basket whose interior defines the chamber 102, 104 is detachably mounted on the base unit 109.

This may facilitate preparation for sterilization of the infant feeding equipment, since the basket, while detached from the base unit 109, can be loaded with infant feeding equipment. Also, in embodiments in which the water holder 107B is included in the base unit 109, water can be dispensed into the water holder 107B while the basket is detached from the base unit 109.

Steam sterilization can then begin once the loaded basket is attached to the base unit 109 whose water holder 107B has been filled with water.

The apparatus 100 includes a vent 108 for venting air and/or steam from the chamber 102, 104, and a valve assembly 110 that restricts venting from the chamber 102, 104 via the vent 108 during steam sterilizing, but allows venting from the chamber 102, 104 via the vent 108 following the steam sterilizing.

The vent 108 may be provided in the chamber wall portion 103.

In some embodiments, such as that shown in FIG. 1, the vent 108 is provided in the lid defining the chamber wall portion 103.

In alternative embodiments, the vent 108 is provided in a wall portion 103 that is included in the housing 101 of the apparatus 100, e.g. the housing 101 to which a lid is moveably, e.g. detachably, mounted.

By the valve assembly 110 allowing venting from the chamber 102, 104 following the steam sterilizing, the steam-sterilized infant feeding equipment may be assisted to dry more rapidly. This is because residual humidity present in the chamber 102, 104 may be vented and/or condensed water on the infant feeding equipment may be allowed to evaporate and escape from the chamber 102, 104 via the vent 108.

However, by the valve assembly 110 restricting, e.g. blocking, venting from the chamber 102, 104 via the vent 108 during the steam sterilizing, more of the steam supplied into the chamber 102, 104 may be retained therein during the steam sterilizing. This may assist to make the steam sterilization more energy efficient.

The valve assembly 110 restricting and allowing venting from the chamber 102, 104 via the vent 108 respectively during and following the steam sterilizing can be implemented in any suitable manner.

Preferably, the valve assembly 110 is arranged at or proximal to the top of the chamber 102, 104.

Alternative positions for the valve assembly 110 can also be contemplated, e.g. in relation to airflow in the chamber 102, 104.

In some embodiments, one or more conditions, e.g. in the chamber 102, 104, following the steam sterilizing is or are different from during the steam sterilizing, and the valve assembly 110 is configured to respond to the one or more different conditions following the steam sterilizing to switch from restricting to allowing of venting from the chamber 102, 104 via the vent 108.

In some embodiments, the atmospheric conditions within the steam chamber 102, 104 cause the valve assembly 110 to switch.

Such atmospheric conditions may, for example, include one or more of temperature, pressure, humidity, and air movement.

Alternatively or additionally, electrical or electromechanical means that are used to create a change in the chamber 102, 104 cause switching of the valve assembly 110.

For example, turning of a blower, e.g. ventilator, shaft may open a valve of the valve assembly 110. The air movement caused by the blower, e.g. ventilator, may be an overlapping point with the above-mentioned air movement-driven valve switching.

Alternatively or additionally, a mechanical switch may switch off the steam and switch on the heating element 107A and/or blower, and this action, e.g. caused by user interaction with the apparatus 100, may cause a valve of the valve assembly 110 to open.

In a simple embodiment, a switch actuated by a user causes opening of a valve of the valve assembly 110.

In some embodiments, a temperature in the chamber 102, 104 during the steam sterilizing is different from, for example higher than, a temperature in the chamber 102, 104 following the steam sterilizing. In such embodiments, the valve assembly 110 may be configured to respond to the temperature in the chamber 102, 104 to switch between restricting and allowing of venting from the chamber 102, 104 via the vent 108.

Thus, the different, e.g. lower, temperature employed following the steam sterilizing may cause the valve assembly 110 to switch from restricting venting from the chamber 102, 104 via the vent 108 to allowing venting from the chamber 102, 104 via the vent 108.

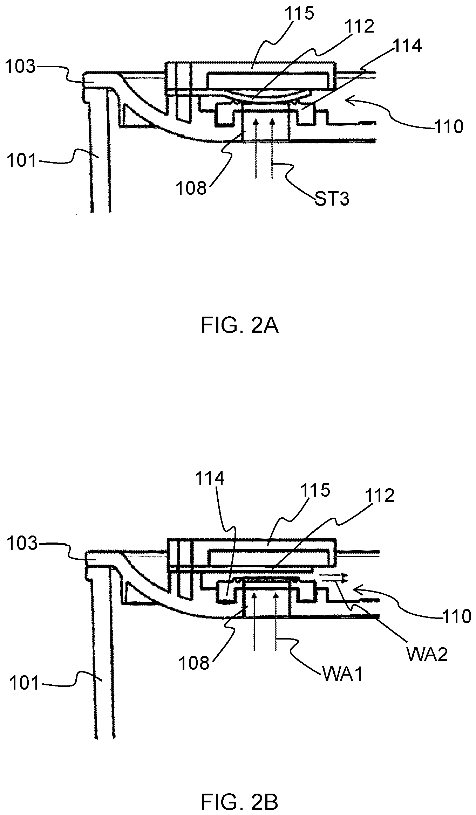

In some embodiments, such as that shown in FIGs. 1, 2A and 2B, the valve assembly 110 comprises a thermally responsive deformable member 112 whose thermally responsive deformation enables said switching between restricting and allowing of venting from the chamber 102, 104 via the vent 108.

Referring to FIG. 2A, the thermally responsive deformable member 112 is depicted restricting, e.g. blocking, venting of steam ST3 from the chamber 102, 104 via the vent 108. In this non-limiting example, the thermally responsive deformable member 112 is arranged to deform against a valve seat 114 that surrounds the vent 108 in response to the temperature of the steam ST3. This deformation of the thermally responsive deformable member 112 against the valve seat 114 causes restricting of venting from the chamber 102, 104 via the vent 108.

In some embodiments, the valve seat 114 comprises, or is in the form of, a sealing member, such as an elastomeric sealing member.

Such a sealing member, e.g. elastomeric sealing member, may assist to retain steam within the chamber 102, 104 during the steam sterilizing.

The sealing member may be formed of any suitable material, such as silicone rubber.

Referring to FIG. 2B, the thermally responsive deformable member 112 is depicted allowing venting of warm air and steam WA1 from the chamber 102, 104 via the vent 108. In this non-limiting example, the thermally responsive deformable member 112 is displaced from the valve seat 114 that surrounds the vent 108 in response to the temperature of the warm air and steam WA1 being lower than that of the steam ST3 during the steam sterilizing. This displacement of the thermally responsive deformable member 112 causes allowance of venting from the chamber 102, 104 via the vent 108, as denoted by the arrows WA2 in FIG. 2B.

More generally, the thermally responsive deformable member 112 may releasably engage the valve seat 114, e.g. elastomeric sealing member, to restrict and allow venting from the chamber 102, 104 via the vent 108.

In at least some embodiments, the deformation of the thermally responsive deformable member 112 is reversible such as to enable repeated switching between restricting and allowing of venting from the chamber 102, 104 via the vent 108.

In the non-limiting example shown in FIGs. 2A and 2B, the deformation of the thermally responsive deformable member 112 is such that the thermally responsive deformable member 112 adopts a domed shape in response to the temperature of the steam ST3 during the steam sterilizing, and reverts to a planar shape in response to the different, e.g. lower, temperature of the warm air and steam WA1 following the steam sterilizing.

Adoption of the domed shape results in the thermally responsive deformable member 112 engaging the valve seat 114 to restrict, e.g. block, venting of the steam ST3 from the chamber 102, 104 via the vent 108. Adoption of the planar shape results in the thermally responsive deformable member 112 disengaging from the valve seat 114 to allow venting of the warm air and steam from the chamber 102, 104 via the vent 108.

Any suitable type of thermally responsive deformable member 112 can be contemplated provided that the thermally responsive deformable member 112 is capable of deforming, e.g. reversibly deforming, to enable switching between restricting and allowing of venting from the chamber 102, 104 via the vent 108.

In some embodiments, the thermally responsive deformable member 112 comprises a layered strip having a first layer and a second layer arranged on the first layer, with a coefficient of thermal expansion of the first layer being different to that of the second layer such that the layered strip provides the thermally responsive deformation.

The layered strip can, for example, comprise or be a bimetallic strip.

Such a bimetallic strip may have the advantage of exhibiting relatively large deformation, particularly in a relatively small area, in response to temperature change.

The bimetallic strip may comprise two kinds of metals (or metal alloys) laid to oppose each other. These two metals may have differences in expansion and shrinkage rate with temperature change. When an intended temperature is reached, the bimetallic strip may activate to change its shape in order to open or close the vent 108.

In some embodiments, and with continued reference to FIGs. 2A and 2B, the apparatus 100 includes a cover member 115 arranged over the thermally responsive deformable member 112, e.g. layered strip, with a passage for the released air and/or steam WA2 being defined between the cover member 115 and an exterior surface of the chamber wall portion 103. In such embodiments, the exterior surface of the chamber wall portion 103 is facing away from, and is not exposed to, the chamber 102, 104.

Such a cover member 115 may assist to protect the thermally responsive deformable member 112, e.g. bimetallic strip, from being polluted, for instance by dust.

The steam sterilizing may be implemented by the apparatus 100 operating in a steam sterilizing mode in which steam is supplied into the chamber 102, 104, e.g. by the steam generator mounted within the housing 101 of the apparatus 100.

An equipment drying mode of the apparatus 100 may be for drying the steam-sterilized infant feeding equipment following, e.g. following termination of, the steam sterilizing mode.

The equipment drying mode may, for example, comprise the supply of steam into the chamber 102, 104 being restricted or ceased.

In at least some embodiments, such as that shown in FIG. 1, the apparatus 100 includes a blower 116 configured to, while the valve assembly 110 is allowing venting from the chamber 102, 104 via the vent 108, blow steam and/or air out of the chamber 102, 104 via the vent 108.

The blower 116 may assist to dry the infant feeding equipment following the steam sterilization.

In such embodiments, the equipment drying mode may comprise operating the blower 116 to blow steam and/or air out of the chamber 102, 104 via the vent 108.

The blower 116 may have any suitable design. In some embodiments, such as that shown in FIG. 1, the blower 116 comprises a motor and a fan whose rotation, to displace steam and/or air out of the chamber 102, 104 via the vent 108, is driven by the motor.

In some embodiments, such as that shown in FIG. 1, the blower 116 is included, e.g. mounted, in the base unit 109, for instance together with the heating element 107A.

In some embodiments, the valve assembly 110 is configured to, responsive to operation of the blower 116, allow venting from the chamber 102, 104 via the vent 108.

Thus, operation of the blower 116 may trigger the valve assembly 110 to allow venting of air and/or steam from the chamber 102, 104 via the vent 108.

In a non-limiting illustrative example, the steam generator in the base unit 109 may generate hot steam that is delivered into the chamber 102, 104, e.g. via the opening(s) of the perforate wall 105B included in the basket, to sterilize the infant feeding equipment. The hot steam may initially pass through the vent 108, in other words exhaust hole, provided in the chamber wall portion 103, e.g. top lid, and heat the thermally responsive deformable member 112, e.g. bimetallic strip.

Once the thermally responsive deformable member 112 has reached the intended temperature, the thermally responsive deformable member 112 may change shape from the planar to the domed shape, and thereby cover the vent 108. The sealing member, e.g. seal ring, around the vent 108 may work together with the thermally responsive deformable member 112 to close the vent 108 and thus reduce hot air and/or steam leakage from the vent 108. In this manner, the chamber 102, 104, e.g. the interior of the basket, may be kept hot and the heating time for sterilizing may be correspondingly reduced, so as to increase energy efficiency.

Once the sterilizing process has finished, drying of the infant feeding equipment, in other words a drying function, may be implemented in which the blower 116, e.g. air fan, blows warm air into the chamber 102, 104 to facilitate drying of the infant feeding equipment. When the airflow reaches the thermally responsive deformable member 112, e.g. bimetallic strip, the surface temperature of the thermally responsive deformable member 112 may be reduced. When the thermally responsive deformable member 112 cools down to the intended value, the shape of the thermally responsive deformable member 112 may change back from the domed shape to the planar shape. The latter may cause the thermally responsive deformable member 112 to rise away from the sealing member. The thus provided clearance between the thermally responsive deformable member 112 and the sealing member may open the vent 108 to allow steam to escape from the chamber 102, 104, thereby increasing the speed of drying of the infant feeding equipment.

With this self-adjustment, in other words automatic, venting control the time for sterilizing and drying may be optimized, and the apparatus 100 may consume less energy.

In embodiments in which the apparatus 100 includes the blower 116, a pressure in the chamber 102, 104 during the steam sterilizing may be different from, e.g. lower than, a pressure in the chamber 102, 104 following the steam sterilizing due to operation of the blower 116 to dry the infant feeding equipment (but the blower 116 not being operated during the steam sterilizing).

In such embodiments, the valve assembly 110 may be configured to respond to the pressure in the chamber 102, 104 to switch between restricting and allowing of venting from the chamber 102, 104 via the vent 108.

Alternatively or additionally, the valve assembly 110 may be configured to respond to an airflow in the chamber 102, 104, e.g. an airflow direction, position and/or magnitude, that is different following the sterilizing as compared to during the sterilizing to switch between restricting and allowing of venting from the chamber 102, 104 via the vent 108.

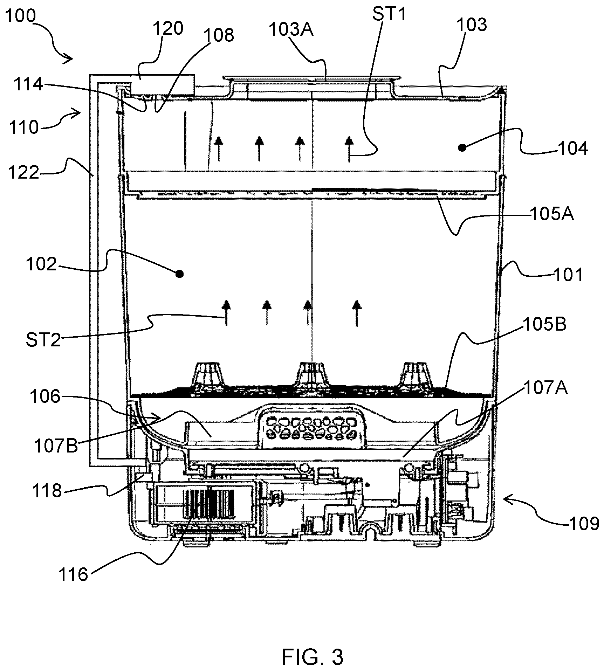

In some embodiments, such as that shown in FIG. 3, the apparatus 100 includes a switching assembly 118 for switching the apparatus between operation in the steam sterilizing mode and operation in the equipment drying mode in which the supply of steam into the chamber 102, 104 is restricted or ceased.

In such embodiments, the valve assembly 110 may be configured to, in response to switching via the switching assembly, switch between restricting and allowing of venting from the chamber 102, 104 via the vent 108.

Such a switching assembly 118 can, for example, include or be defined by a solenoid, e.g. a solenoid arranged to activate the motor to rotate the fan of the blower 116.

In some embodiments, such as that shown in FIG. 3, the valve assembly 110 comprises a valve member 120 connected to a linking member 122 arranged to transfer movement from the switching assembly 118, e.g. solenoid, to the valve member 120 in order to open and close the vent 108 in response to activation of the motor to rotate the fan of the blower 116.

Alternatively, movement of the motor may cause the valve assembly 110 to switch between restricting and allowing of venting from the chamber 102, 104 via the vent 108.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. An infant feeding equipment-sterilizing apparatus (100), the apparatus comprising:
a chamber (102, 104) for receiving infant feeding equipment to be steam sterilized;
a vent (108) for venting air and/or steam from the chamber; and
a valve assembly (110) switchable between restricting venting from the chamber via the vent during steam sterilizing, and allowing venting from the chamber via the vent following the steam sterilizing.

2. The apparatus (100) according to claim 1, wherein one or more conditions following the steam sterilizing is or are different from during the steam sterilizing, and wherein the valve assembly (110) is configured to respond to the one or more different conditions following the steam sterilizing to switch from restricting to allowing venting from the chamber via the vent (108).

3. The apparatus (100) according to claim 1 or claim 2, wherein electrical or electromechanical means that are used to create a change in the chamber (102, 104) between during and following the steam sterilizing cause switching of the valve assembly (110) from restricting to allowing venting from the chamber via the vent (108).

4. The apparatus (100) according to any one of claims 1 to 3, wherein user actuation of a switch for creating a change in the chamber (102, 104) between during and following the steam sterilizing causes switching of the valve assembly (110) from restricting to allowing venting from the chamber via the vent (108).

5. The apparatus (100) according to any one of claims 1 to 4, wherein the valve assembly (110) comprises a thermally responsive deformable member (112) whose thermally responsive deformation enables said switching between restricting and allowing of venting from the chamber (102, 104) via the vent (108).

6. The apparatus (100) according to claim 5, wherein the thermally responsive deformable member (112) comprises a layered strip having a first layer and a second layer arranged on the first layer, a coefficient of thermal expansion of the first layer being different to that of the second layer such that the layered strip provides said thermally responsive deformation.

7. The apparatus (100) according to any one of claims 1 to 6, comprising a switching assembly (118) for switching the apparatus between operation in a steam sterilizing mode in which steam is supplied into the chamber (102, 104), and operation in an equipment drying mode in which the supply of steam into the chamber is restricted or ceased, wherein the valve assembly is configured to, in response to switching via the switching assembly, switch between restricting and allowing of venting from the chamber via the vent.

8. The apparatus (100) according to any one of claims 1 to 7, comprising a blower (116) configured to, while the valve assembly (110) is allowing venting from the chamber (102, 104) via the vent (108), blow steam and/or air from the chamber via the vent.

9. The apparatus (100) according to claim 8, wherein the valve assembly (110) is configured to, responsive to operation of the blower (116), allow venting from the chamber (102, 104) via the vent (108).

10. The apparatus (100) according to claim 8 or claim 9 as according to claim 7, wherein operation of the apparatus in the equipment drying mode further comprises the blower (116) being operated to blow steam and/or air from the chamber (102, 104) via the vent (108).

11. The apparatus (100) according to any one of claims 1 to 10, comprising a lid openable to access the chamber (102, 104), the vent (108) and at least part of the valve assembly (110) being provided in the lid.

12. The apparatus (100) according to any one of claims 1 to 11, wherein the vent (108) comprises an aperture delimited by a chamber wall portion (103) that at least partly delimits the chamber (102, 104), and the valve assembly (110) comprises a valve seat (114) that extends around the aperture, wherein the valve seat comprises a sealing member.

13. The apparatus (100) according to claim 12 as according to claim 5 or claim 6, wherein the thermally responsive deformable member (112) is configured to releasably engage the sealing member to restrict and allow venting from the chamber (102, 104) via the vent (108).

14. A chamber wall portion (103) for an infant feeding equipment-sterilizing apparatus (100), the chamber wall portion being for at least partly delimiting a chamber (102, 104) in which infant feeding equipment to be steam sterilized is receivable, wherein a vent (108) for venting air and/or steam from the chamber is provided in the chamber wall portion together with at least part of a valve assembly (110) that is switchable between restricting venting from the chamber via the vent during steam sterilizing, and allowing venting from the chamber via the vent following the steam sterilizing.

15. A method for sterilizing infant feeding equipment using an apparatus (100) having a chamber (102, 104), a vent (108) for venting air and/or steam from the chamber, and a valve assembly (110), the method comprising:
receiving infant feeding equipment in the chamber; and
steam sterilizing the infant feeding equipment, wherein venting from the chamber via the vent is restricted by the valve assembly during the steam sterilizing, and is allowed by the valve assembly following the steam sterilizing.
